# FASCICULE DE BREVET EUROPEEN

(11) **EP 3 930 014 B1**
(45) Date de publication et mention de la délivrance du brevet: **02.11.2022**
(21) Numéro de dépôt: 20315317.6
(22) Date de dépôt: 24.06.2020
(51) Int. Cl.: H02N 2/18, H01L 41/113, A61N 1/375, A61N 1/378, H01L 41/047

(54) **MODULE DE RÉCUPÉRATION D'ÉNERGIE À TRANSDUCTEUR PIÉZOÉLECTRIQUE, NOTAMMENT POUR LA RECHARGE OPTIMISÉE DE LA BATTERIE D'UN DISPOSITIF MÉDICAL IMPLANTABLE TEL QU'UNE CAPSULE CARDIAQUE AUTONOME LEADLESS**
ENERGIERÜCKGEWINNUNGSMODUL MIT PIEZOELEKTRISCHEM WANDLER, INSBESONDERE ZUM OPTIMIERTEN WIEDERAUFLADEN DER BATTERIE EINES IMPLANTIERBAREN MEDIZINPRODUKTS, WIE Z. B. EINER KABELLOSEN AUTONOMEN KAPSEL ZUM EINSATZ IM HERZEN
MODULE FOR ENERGY RECOVERY WITH PIEZOELECTRIC TRANSDUCER, IN PARTICULAR FOR OPTIMISED RECHARGING OF THE BATTERY OF AN IMPLANTABLE MEDICAL DEVICE SUCH AS A LEADLESS AUTONOMOUS CARDIAC CAPSULE

(43) Date de publication de la demande: 29.12.2021
(73) Titulaire: Cairdac, 92160 Antony (FR)
(72) Inventeur: Makdissi, Alaa, 75011 Paris (FR); Nguyen-Dinh, An, 37520 La Riche (FR)
(74) Mandataire: Dupuis-Latour, Dominique

(56) Documents cités:
- EP-A1- 3 159 948
- WO-A1-2019/001829
- WO-A2-2007/102937
- US-A1- 2009 174 289
- US-A1- 2015 015 114

## Description

### CONTEXTE DE L'INVENTION

### Domaine de l'invention

L'invention concerne les récupérateurs d'énergie, également dénommés harvesters ou scavengers, qui recueillent l'énergie mécanique résultant de mouvements divers qu'ils subissent et convertissent cette énergie mécanique en énergie électrique.

Elle concerne plus spécifiquement les récupérateurs de type dit "PEH" (Piezoelectric Energy Harvester), qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en œuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

En effet, l'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur d'énergie, également dénommé harvester ou scavenger, pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit.

L'énergie mécanique recueillie par le récupérateur est convertie en énergie électrique (tension ou courant) au moyen d'un transducteur mécanoélectrique approprié, pour assurer l'alimentation des divers circuits et capteurs du dispositif et la recharge de l'organe de stockage d'énergie. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules leadless", pour les distinguer des électrodes ou capteurs disposés à l'extrémité distale d'une sonde (lead) parcourue sur toute sa longueur par un ou plusieurs conducteurs reliés à un générateur connecté à l'extrémité opposée, proximale.

L'invention n'est toutefois pas limitée à un type particulier de capsule, ni même d'implant leadless, et elle est applicable indifféremment à de nombreux autres types de dispositifs médicaux implantables, quelle que soit leur destination fonctionnelle, cardiaque ou autre, médicale ou non. Dans le cas d'une application cardiaque, la capsule leadless surveille en continu le rythme du patient et délivre si nécessaire au cœur des impulsions électriques de stimulation, de resynchronisation et/ou de défibrillation en cas de troubles du rythme détecté par la capsule. La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le WO 2019/001829 A1 (Cairdac) décrit un exemple d'une telle capsule leadless intracardiaque.

L'invention concerne plus précisément les capsules ou dispositifs implantables analogues dont le récupérateur d'énergie est du type PEH, c'est-à-dire utilisant un transducteur piézoélectrique ou "PZT" *(PieZoelectric Transducer)* et un ensemble pendulaire inertiel soumis aux sollicitations externes décrites plus haut. L'ensemble pendulaire inertiel comprend une masse mobile logée dans le corps de la capsule, dite "masse sismique" ou "masse inertielle", qui est entraînée au gré des mouvements de la capsule soumise en permanence aux diverses sollicitations externes décrites plus haut. Après chacune de ces sollicitations, la masse inertielle, qui est couplée à un élément élastiquement déformable, oscille à une fréquence propre d'oscillation libre.

L'énergie mécanique de l'oscillation est convertie en énergie électrique par un transducteur mécanoélectrique produisant un signal électrique. Ce transducteur mécanoélectrique peut en particulier être un PZT sollicité de façon cyclique et alternative en flexion de manière à engendrer au sein du matériau qui le constitue des charges électriques qui sont récupérées en surface du composant pour être utilisées par le système d'autoalimentation de la capsule leadless. Le PZT peut notamment se présenter sous la forme d'une lame encastrée à l'une de ses extrémités et couplée à la masse inertielle à son autre extrémité, qui est libre.

Le signal électrique en sortie du transducteur est délivré à un circuit de gestion de l'alimentation de la capsule, qui redresse et régule le signal électrique pour délivrer en sortie une tension ou un courant continus stabilisés permettant d'assurer l'alimentation des divers circuits électroniques et capteurs de la capsule, ainsi que la recharge de l'organe de stockage d'énergie.

Un tel récupérateur d'énergie de type PEH est notamment décrit dans le US 3,456,134 A (Ko) et dans le WO 2019/001829 A1 précité. Le US 2015/015114 A1 (Hall et al.) décrit également un harvester de type PEH dans lequel un substrat porte une pluralité de transducteurs PZT distincts allongés et parallèles, disposés dans le même sens que la direction principale de flexion du substrat ou bien perpendiculairement à celle-ci, et qui peuvent être commutés en fonction de l'amplitude de la vibration de la lame ou des différents modes de vibration de celle-ci, pour former à volonté un réseau de transducteurs série ou bien parallèle.

On notera que le terme de "lame" doit être entendu dans son sens le plus large, à savoir une bande mince et plate de forme allongée, étant entendu que la forme de cette bande n'est pas nécessairement rectangulaire ni son épaisseur constante (comme dans la description du mode de réalisation particulier qui sera donnée plus bas). Le terme de "lame" au sens de la présente invention recouvre ainsi des éléments pouvant présenter une largeur et/ou une épaisseur qui ne sont pas constantes en direction longitudinale, ainsi qu'éventuellement une déformabilité pouvant aller au-delà d'un unique degré de liberté en flexion.

Le signal électrique produit par la lame piézoélectrique ne peut toutefois pas être directement utilisé pour alimenter les divers circuits électroniques et capteurs de la capsule et recharger l'organe de stockage d'énergie (batterie rechargeable ou autre moyen de stockage tel qu'un condensateur à hautes performances, ci-après désignés par le terme générique de "batterie").

Pour cela, le signal électrique oscillant amorti recueilli aux bornes de la lame piézoélectrique est délivré en entrée à un circuit de gestion de l'alimentation de la capsule, habituellement désigné "PMU" *(Power* *Management Unit).* Ce circuit redresse et régule le signal électrique oscillant et délivre en sortie une tension ou un courant stabilisés permettant d'assurer l'alimentation des circuits électriques de l'implant et la recharge de la batterie.

L'utilisation d'un PZT comme source d'énergie rend cette gestion de l'alimentation particulièrement complexe.

En effet, du point de vue électrique le PZT peut être assimilé à une source de courant alternatif d'amplitude variable ayant une impédance interne élevée, essentiellement capacitive. De fait, pour optimiser l'extraction d'énergie en minimisant les pertes, le PMU doit pouvoir suivre dynamiquement la variation de la tension aux bornes du PZT et décharger de la façon la plus appropriée les charges accumulées dans la capacité interne afin de minimiser les pertes par autodécharge au sein du PZT. D'autre part, après redressement le courant de recharge de la batterie doit être contrôlé en fonction de la tension présente aux bornes de la batterie, cette tension variant en fonction du niveau de la batterie et de la charge électrique instantanée représentant la consommation des circuits électriques de l'implant. Concrètement, la puissance disponible en sortie du PMU pour la recharge peut être représentée par une caractéristique puissance/tension croissante pour des valeurs faibles de la tension de batterie, passant par un maximum, puis décroissant pour des valeurs élevées de la tension de la batterie, la tension produite par le PZT devenant alors plus faible que la tension aux bornes de la batterie et ne permettant plus la recharge de cette dernière.

Ce problème, qui est spécifique à l'emploi d'un PZT comme source primaire d'énergie pour la recharge d'une batterie, rend très délicate la gestion de l'énergie, de sorte que le recours à un circuit dédié complexe de type ASIC est nécessaire pour assurer les fonctions du PMU.

Un type d'ASIC particulièrement adapté met en œuvre des circuits de type SSHI *(Synchronized Switch Harvesting on Inductor,* récupération par commutation synchronisée sur inductance). Ces circuits SSHI sont efficaces mais, du fait de leur complexité, entraînent une consommation non négligeable, qui vient donc au détriment de l'autonomie de l'implant. De plus, dans le cas d'un circuit SSHI, la présence d'une inductance dans le PMU rend celui-ci sensible aux champs magnétiques élevés subis par le patient, et donc par l'implant, lors des examens en imagerie par résonance magnétique (IRM/MRI), avec des risques de perturbation du fonctionnement du circuit, voire de dégradation de celui-ci du fait de surintensités ou d'échauffements internes. De plus, la taille d'une inductance présentant un facteur de qualité élevé n'est pas compatible avec l'extrême miniaturisation requise par une capsule leadless.

L'un des buts de l'invention est de proposer une configuration de PEH qui pallie ces multiples inconvénients et contraintes,
avec un PMU simplifié à l'extrême, dépourvu de tout composant inductif,
dont la consommation propre soit négligeable, et
qui, de plus et surtout, maximise la récupération d'énergie sur une très large plage de tensions de fonctionnement de la batterie.

### RÉSUMÉ DE L'INVENTION

À cet effet, l'invention propose un module de récupération d'énergie de type PEH comprenant, de manière en elle-même connue, notamment d'après le US 2015/015114 A1 précité, les éléments énoncés dans le préambule de la revendication 1.

La partie caractérisante de la revendication 1 énonce les éléments propres à l'invention permettant d'atteindre les buts précités.

Les sous-revendications visent des modes de mise en œuvre subsidiaires avantageux de l'invention.

### DESCRIPTION SOMMAIRE DES DESSINS

On va maintenant décrire un exemple de réalisation de la présente invention en référence aux dessins annexés où les mêmes références désignent d'une figure à l'autre des éléments identiques ou fonctionnellement semblables.
La Figure 1 illustre un dispositif médical de type capsule leadless dans son environnement, implanté au fond du ventricule droit d'un patient.
La Figure 2 présente sous forme schématique les principaux blocs fonctionnels constitutifs de la capsule leadless.
La Figure 3 est un exemple de signal oscillant amorti délivré par le PEH au cours d'un cycle cardiaque.
La Figure 4 est une vue de côté de la capsule de l'invention, avec les principaux éléments mécaniques de l'ensemble pendulaire du PEH.
La Figure 5 est une vue en coupe montrant, avec une échelle des épaisseurs volontairement exagérée, la structure des différentes couches d'un PZT bimorphe utilisable avec la présente invention.
Les Figures 6 et 7 sont des vues en plan, respectivement, du recto et du verso d'un PZT utilisable pour la mise en œuvre de la présente invention, avec une lame munie d'électrodes de surface fractionnées.
Les Figures 8, 9 et 10 illustrent trois configurations électriques équivalentes possibles, respectivement en parallèle, en série et en série-parallèle, selon différents choix de commutation possible des électrodes du PZT.
La Figure 11 illustre trois caractéristiques décrivant la variation de la puissance efficace recueillie par le PEH en fonction de la tension aux bornes de la batterie, pour chacune des trois configurations des Figures 8, 9 et 10.
La Figure 12 est un schéma synoptique de la matrice de commutation permettant de sélectionner les différentes configurations des Figures 8, 9 et 10.
La Figure 13 est un exemple de commutateur statique à MOS complémentaires utilisable pour l'implémentation de la matrice de commutation de la Figure 12.
La Figure 14 illustre, vue en plan, une variante du PZT des Figures 6 et 7 où chaque électrode de surface fractionnée de la lame est elle-même divisée en sous-électrodes électriquement indépendantes, permettant d'augmenter encore les combinaisons de couplage par la matrice de commutation.
La Figure 15 est un schéma synoptique d'une matrice de commutation adaptée au PZT à sous-électrodes multiples de la Figure 14.
Les Figures 16 et 17 illustrent deux configurations électriques équivalentes additionnelles rendues possibles grâce au PZT à électrodes multiples de la Figure 14.
La Figure 18 illustre six caractéristiques décrivant la variation de la puissance efficace recueillie par le PEH en fonction de la tension aux bornes de la batterie, pour diverses configurations susceptibles d'être sélectionnées par la matrice de la Figure 15.

### DESCRIPTION DÉTAILLÉE D'UN MODE DE RÉALISATION PRÉFÉRENTIEL DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

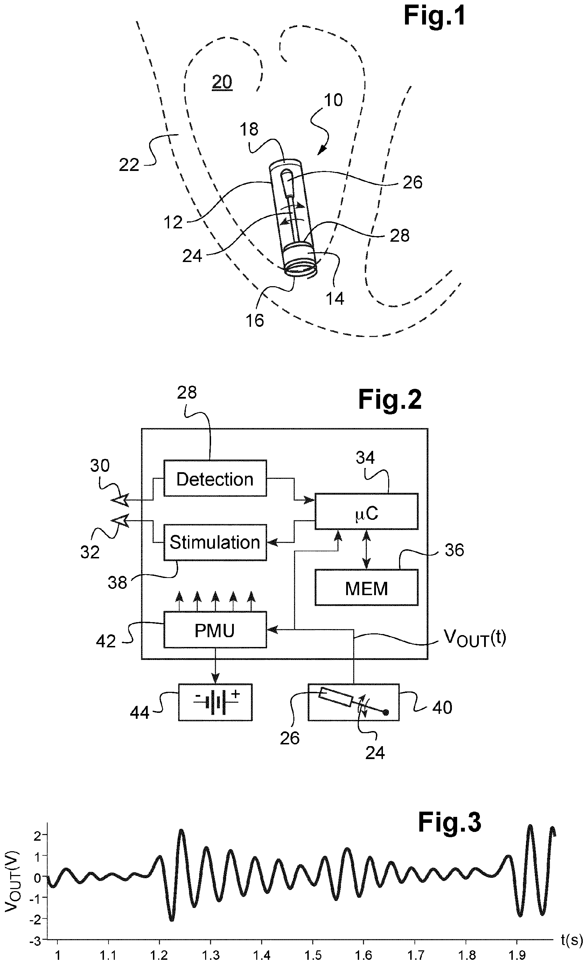

Sur la Figure 1, on a représenté un dispositif de type capsule leadless 10 dans une application à la stimulation cardiaque.

La capsule 10 se présente sous forme extérieure d'un implant avec un corps tubulaire allongé cylindrique 12 enfermant les divers circuits électroniques et d'alimentation de la capsule ainsi qu'un récupérateur d'énergie à ensemble pendulaire. Les dimensions typiques d'une telle capsule sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en œuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 1, la capsule leadless 10 est un implant endocavitaire implanté à l'intérieur d'une cavité 20 du myocarde 22, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en œuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

L'ensemble pendulaire est constitué d'une lame piézoélectrique 24 encastrée en 28 à l'une de ses extrémités et dont l'extrémité opposée, libre, est couplée à une masse inertielle mobile 26. La lame piézoélectrique 24 est une lame flexible élastiquement déformable qui constitue avec la masse inertielle 26 un système pendulaire de type masse-ressort. Du fait de son inertie, la masse 26 soumet la lame 24 à une déformation de type vibratoire de part et d'autre d'une position neutre ou non déformée correspondant à une position stable de repos en l'absence de toute sollicitation.

De fait, pour ce qui est de son comportement mécanique, cet ensemble peut être assimilé à une structure de type "poutre encastrée-libre", présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondant à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 24 assure en outre, par effet piézoélectrique, une fonction de transducteur mécanoélectrique permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les divers circuits électroniques de la capsule.

La lame est avantageusement une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT ou les monocristaux de type PMN-PT, titanate de baryum ou niobate de lithium. La Figure 2 est un synoptique des divers circuits électriques et électroniques intégrés à la capsule leadless, présenté sous forme de blocs fonctionnels.

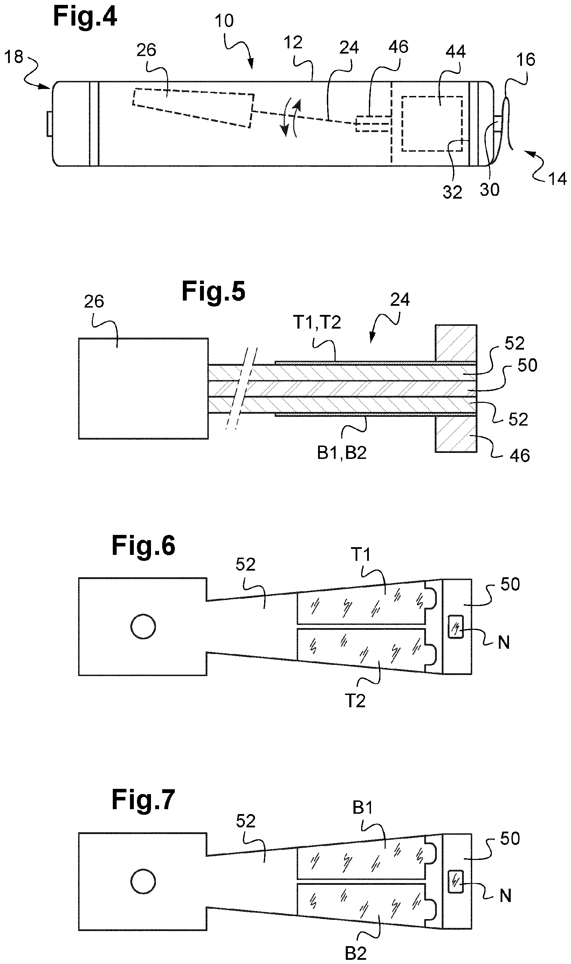

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule (voir Figure 4). Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer en tant que de besoin au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 24 et la masse inertielle 30 décrits plus haut en référence à la Figure 1.

Comme la lame piézoélectrique 24 assure aussi une fonction de transducteur mécano-électrique, elle convertit en charges électriques les sollicitations mécaniques subies et produit un signal électrique variable V_{OUT}(t) dont les variations sont illustrées Figure 3, qui est un signal alternatif oscillant à la fréquence d'oscillation libre de l'ensemble pendulaire lame 24/masse 30, et au rythme des battements successifs du myocarde auquel la capsule est couplée.

Le signal électrique variable V_{OUT}(t) est délivré à un circuit de gestion de l'énergie ou PMU 42. Le PMU 42 redresse et régule le signal V_{OUT}(t) de manière à produire en sortie une tension ou un courant continus stabilisés servant à l'alimentation des divers circuits électroniques et à la recharge de la batterie intégrée 44.

La Figure 4 illustre l'agencement mécanique des différents composants que l'on vient de décrire à l'intérieur du corps cylindrique 12 de la capsule leadless 10, avec notamment la lame 24 dont l'une des extrémités, libre, est solidaire de la masse inertielle 26, et dont l'extrémité opposée est assujettie au corps de la capsule par un encastrement 46, l'ensemble pouvant être assimilé, comme expliqué plus haut, à une structure de type "poutre encastrée-libre".

La Figure 5 illustre de façon schématique, avec une échelle des épaisseurs volontairement exagérée, la structure des différentes couches d'une lame piézoélectrique bimorphe utilisable pour la mise en œuvre de la présente invention, avec les deux côtés recto et verso de la lame illustrés isolément sur les vues en plan respectives des Figures 6 et 7.

La lame 24 utilisée est une lame de type bimorphe, c'est-à-dire comprenant deux couches 52 de matériau céramique PZT déposées sur chacune des faces opposées d'une âme centrale ou "shim" 50 en matériau conducteur (ou en variante en un matériau isolant, avec des ponts de contact permettant de shunter les électrodes internes des couches piézoélectriques). Cette structure bimorphe correspond en fait à l'association de deux structures unimorphes placées dos-à-dos, avec mise en commun de l'âme supportant le matériau PZT. D'autre part, dans le cas de la présente invention, l'âme 50 est une âme en matériau conducteur, de sorte qu'il est possible de recueillir les charges produites par la déformation du matériau PZT aussi bien entre l'âme conductrice et une électrode de surface de l'un et/ou de l'autre des PZT, qu'entre les deux électrodes de surface des faces opposées de la lame, indépendamment de l'âme centrale.

Les Figures 6 et 7 sont des vues en plan respectivement du recto et du verso d'un PZT utilisable pour la mise en œuvre de la présente invention, avec une lame munie d'électrodes de surface fractionnées.

Afin de renforcer la fiabilité de la lame 24, sa forme en plan est avantageusement, comme illustré, une forme trapézoïdale avec une décroissance (linéaire ou exponentielle) de la largeur pour mieux répartir les contraintes le long de la lame, ces contraintes étant plus fortes à proximité et au niveau de l'encastrement 46, et nulles au niveau de la masse inertielle 26. De plus, la forme trapézoïdale permet d'ajuster la fréquence de résonance de l'ensemble en fonction de la géométrie du trapèze, tout en maximisant l'amplitude de déplacement de la masse du fait que l'extrémité libre est plus étroite que l'extrémité encastrée. Avantageusement, et de façon caractéristique de l'invention, l'une et/ou l'autre des électrodes de surface (dans l'exemple illustré les deux électrodes de surface) sont fractionnées en deux sous-électrodes électriquement isolées entre elles, à savoir les électrodes T1 et T2 sur la face supérieure *(top)* et, de même, B1 et B2 sur la face inférieure *(bottom).* Ces quatre sous-électrodes comportent chacune une plage respective pour la prise de contact et la liaison aux circuits du PMU 42 ; l'âme centrale 50 comporte également une plage de contact correspondante N, utilisée comme potentiel de référence électrique de "neutre".

On notera que sur les figures chaque électrode de surface est fractionnée en deux sous-électrodes sensiblement symétriques par rapport à un axe longitudinal central de la lame. Cette configuration ne doit toutefois être en aucun cas considérée comme limitative, de nombreuses autres configurations de fractionnement étant aussi bien envisageables, de même qu'un fractionnement de chaque électrode, ou de chacune des électrodes, en plus de deux sous-électrodes, ou encore le fractionnement d'une seule des deux électrodes.

La lame comporte ainsi cinq points de liaison électrique distincts T1, T2, B1, B2 et N.

L'idée de base de l'invention consiste à définir à partir de ces divers points de liaison une pluralité de paires d'électrodes entre lesquelles peuvent être recueillis simultanément une pluralité correspondante de signaux oscillants résultant des charges produites par le matériau PZT des deux couches 52, et à commuter entre elles dynamiquement ces différentes paires d'électrodes de la pluralité de paires d'électrodes de manière à définir plusieurs configurations possibles, résultant de couplages différents de ces paires d'électrodes entre elles, pour délivrer en sortie un signal oscillant combiné appliqué au PMU 42.

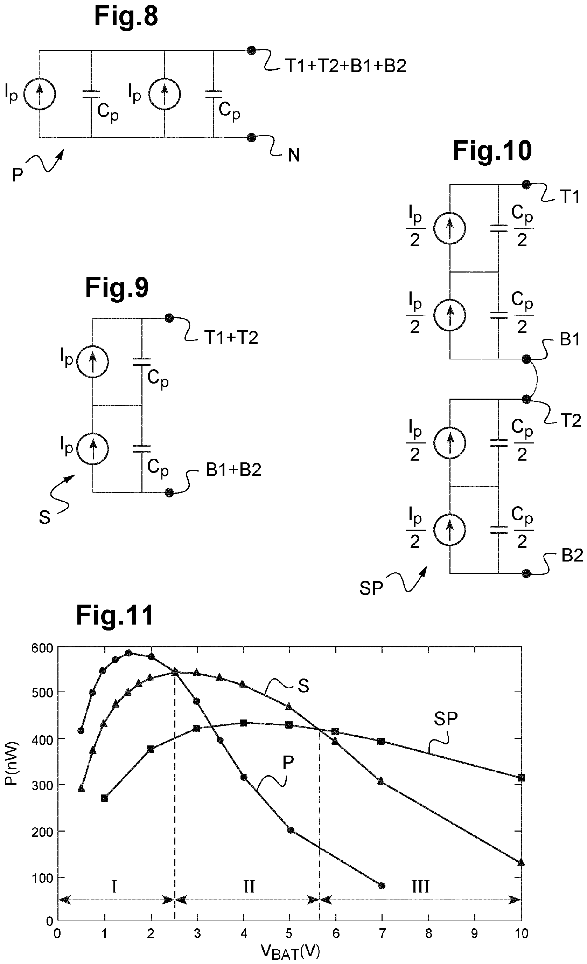

Les Figures 8, 9 et 10 illustrent ainsi trois configurations possibles, respectivement parallèle, série et série-parallèle qu'il est ainsi possible d'obtenir en modifiant le couplage des électrodes entre elles.

Pour ces différentes figures, Iₚ représente le courant délivré par chaque couche 52 de PZT, assimilé à un générateur de courant, et Cₚ la capacité interne de ce générateur de courant.

Dans la configuration parallèle P illustrée Figure 8, les deux sources de courant constituées par chacune des deux couches 52 de PZT sont mises en parallèle, de même que leurs capacités internes. Cette configuration parallèle P est équivalente à une source de courant 2.Iₚ, donc double de celle d'une lame unimorphe n'ayant qu'une seule couche 52 de PZT sur l'âme 50), mais en revanche avec une tension produite deux fois plus faible que celle d'une lame unimorphe en raison du doublement de la capacité interne.

Dans la configuration série S de la Figure 9, la liaison à l'âme n'est pas utilisée et les deux couches de PZT 52 sont en série. Ceci donne une source de courant Iₚ, identique à ce que produirait une lame unimorphe ; en revanche, les deux capacités Cₚ étant en série, la capacité équivalente est divisée par deux et donc la tension en sortie est deux fois plus élevée que celle d'une lame unimorphe.

La configuration série-parallèle illustrée Figure 10 utilise le fractionnement des électrodes de surface en sous-électrodes T1, T2 respectivement B1, B2 illustrées Figures 6 et 7. Cette configuration équivaut à une source de courant Iₚ/2 ayant une capacité interne Cₚ/8. Par rapport à une lame unimorphe, le courant produit est deux fois moindre mais la tension est huit fois supérieure du fait de la réduction de la capacité interne.

En ce qui concerne la puissance délivrée, on démontre que celle-ci est la même dans les trois configurations illustrées, et qu'elle vaut le double de la puissance que délivrerait une lame unimorphe.

En d'autres termes, ni le fractionnement des électrodes en sous-électrodes, ni le type de configuration de couplage utilisée (série, parallèle, ou série-parallèle) ne modifie la puissance efficace maximale théorique que peut produire le PEH. Seules changent la valeur du courant délivré par le PEH et celle de la capacité interne de la source de courant, donc de la tension en sortie du PEH.

La Figure 11 illustre trois caractéristiques décrivant la variation de la puissance efficace produite par le PEH en fonction de la tension V_{BAT} aux bornes de la batterie, pour chacune des trois configurations série, parallèle, ou série-parallèle des Figures 8, 9 et 10.

Comme on peut le voir, pour chacune des configurations S, P et SP, la courbe passe par un maximum puis décroit progressivement, le maximum de puissance correspondant à la condition R_{L} = 1/ωCₚ, R_{L} étant l'impédance de la charge sur laquelle est délivrée la puissance et ω étant la pulsation de résonance de l'ensemble pendulaire.

Sur une charge idéale, résistive, la puissance optimale serait, comme expliqué plus haut, la même quelle que soit la configuration choisie. Concrètement, cette puissance est toutefois affectée par diverses pertes de charge (courant de fuite des diodes, etc.) ainsi que par diverses non-linéarités dues notamment au fait que la charge modifie le régime d'oscillation du PEH par un effet d'amortissement aux tensions de sortie les plus élevées.

Avantageusement et de façon caractéristique de l'invention, le mode de couplage des différentes paires d'électrodes (parallèle, série ou série-parallèle) est sélectionné en fonction du niveau V_{BAT} mesuré de la tension de batterie, de manière à sélectionner celles des caractéristiques P, S ou SP qui est susceptible de maximiser la puissance délivrée par le PEH. Dans l'exemple des caractéristiques de la Figure 11, on peut ainsi définir trois plages I, Il et III :
Plage I : pour les tensions de batterie les plus basses, la configuration parallèle P est sélectionnée,
Plage II : pour les tensions intermédiaires, la configuration série S est sélectionnée,
Plage III : pour les tensions les plus élevées, la configuration série-parallèle SP est sélectionnée.

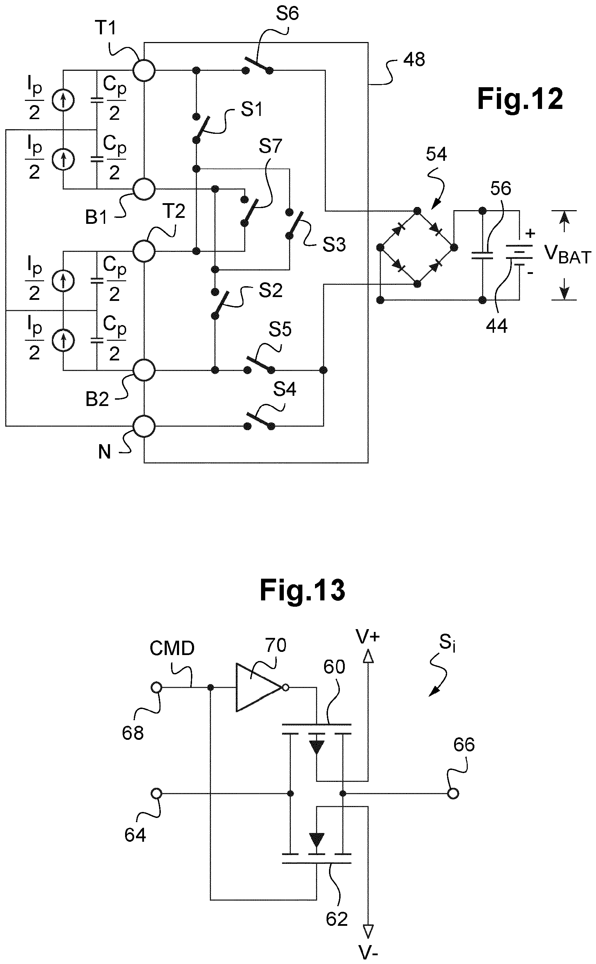

La Figure 12 est un schéma synoptique de la matrice de commutation permettant de sélectionner les différentes configurations des Figures 8, 9 et 10.

Cette matrice de commutation 48 est couplée aux différentes électrodes T1, T2, B1, B2 et N décrites plus haut. Elle combine entre elles ces différentes électrodes au moyen de commutateurs S1... S7 et délivre en sortie un signal (oscillant) combiné, à un PMU de recharge de la batterie qui, comme dans l'exemple illustré, peut être réduit à sa plus simple expression, avec un redresseur double alternance ou FBR *(Full-wave Bridge Rectifier)* 54, par exemple un pont de Graetz, et un condensateur de filtrage 56 couplés à la batterie 44.

L'état ouvert ou fermé des divers commutateurs S1... S7 permet de reconstituer sélectivement les configurations parallèle-série ou parallèle illustrées Figure 10. Ainsi :
pour la configuration série S, les commutateurs à fermer sont : S1 (pour coupler T1 avec T2 et former une seule électrode *top* T=T1+T2), S2 (pour coupler B1 avec B2 et former une seule électrode *bottom* B=B1+B2), et S5 et S6 (pour connecter respectivement B et T au pont redresseur 54) ;
pour la configuration parallèle P, les commutateurs à fermer sont : S1 (pour coupler T1 avec T2 et former une seule électrode *top* T=T1+T2, S2 (pour coupler B1 avec B2 et former une seule électrode *bottom* B=B1+B2), S3 (pour connecter en parallèle T et B et former une seule référence électrique T/B), S6 (pour connecter T/B à une extrémité du pont redresseur) et S4 (pour connecter l'autre extrémité du pont redresseur ) à l'électrode neutre N du *shim* 50) ;
Pour la configuration série-parallèle SP, utilisant le fractionnement des électrodes T1/T2 et B1/B2, les commutateurs à fermer sont : S5, S6 et S7.

Le pont redresseur 54 commencera à charger la batterie lorsque la tension en circuit ouvert du signal délivré en sortie de la matrice de commutation 48 sera supérieure à V_{BAT} + 2V_{D} (V_{D} étant la chute de tension égale à la tension de seuil des diodes du pont 54).

L'ouverture ou la fermeture de chacun de commutateurs S1... S7 est commandée en fonction du niveau de tension V_{BAT} de la batterie :
si V_{BAT} est compris dans la plage I de la Figure 11, alors on fermera les commutateurs qui sélectionnent la configuration parallèle P ;
si V_{BAT} est compris dans la plage II, on sélectionnera la configuration série S ;
et pour les tensions supérieures de V_{BAT}, correspondant à la plage III, on sélectionnera la configuration série-parallèle SP.

On notera que, la tension V_{BAT} variant de façon lente, les transitions d'une plage à l'autre, et donc les changements de configuration P, S ou SP, seront relativement peu fréquents.

Du point de vue de l'implémentation matérielle, il est avantageux d'utiliser pour les commutateurs S1... S7 de la matrice des commutateurs statiques dont l'essentiel de la consommation (par ailleurs minime) résulte des changements d'état passant/bloqué.

La Figure 13 illustre un exemple d'un tel commutateur statique à transistors MOS complémentaires utilisable pour l'implémentation de la matrice de commutation 48 de la Figure 12

Chaque commutateur comporte une borne d'entrée 64 et une borne de sortie 66 couplées par deux transistors PMOS 60 et NMOS 62 complémentaires commandés par un signal CMD appliqué sur une borne 68 et inversé pour l'un des transistors par un inverseur 70. Le signal de commande CMD peut être généré par exemple par le microcontrôleur 34, dès lors que son niveau est supérieur ou égal à celui de la tension présente sur les bornes 64 et 66.

La consommation d'une telle matrice de commutation 48 est quasiment nulle, à la différence des circuits conventionnels utilisés généralement par les PEH, mettant en œuvre des ASIC très complexes tels que ceux du type SSHI évoqué en introduction.

La simplification du circuit permet en outre un gain de surface important sur la carte de circuit imprimé de la capsule leadless, le PMU étant, comme on le voit, réduit grâce à l'invention à sa plus simple expression sur le plan des composants à utiliser.

Du point de vue du fonctionnement dynamique, pour la production des signaux de commande CMD de chaque commutateur S1... S7 la seule opération consiste à comparer à intervalles réguliers la tension V_{BAT} présente aux bornes de la batterie à deux seuils (ceux séparant respectivement les plages I/II et II/III) pour modifier (éventuellement) la configuration des paires d'électrodes du PEH couplées entre elles.

On notera que dans la présente description on a considéré le cas d'un transducteur comprenant cinq points de liaison électrique distincts T1, T2, B1, B2 et N, couplés de manière à définir trois configurations électriques équivalentes S, P ou SP.

Ce choix n'est toutefois donné qu'à titre d'exemple et ne doit être en aucun cas considéré comme limitatif, de nombreuses autres configurations étant aussi bien envisageables dans le cadre de l'invention, avec un plus ou moins grand nombre de points de liaison et/ou un nombre de configurations électriques équivalentes moindre (par exemple seulement les configurations S et P, sans qu'il soit nécessaire de fractionner les électrodes), ou supérieur (par exemple quatre ou cinq configurations, mettant en œuvre un plus grand nombre de points de liaison), dès lors que ces différentes configurations sont commutables dynamiquement et automatiquement sous le contrôle du processeur de l'implant en fonction d'un ou plusieurs critères prédéterminés.

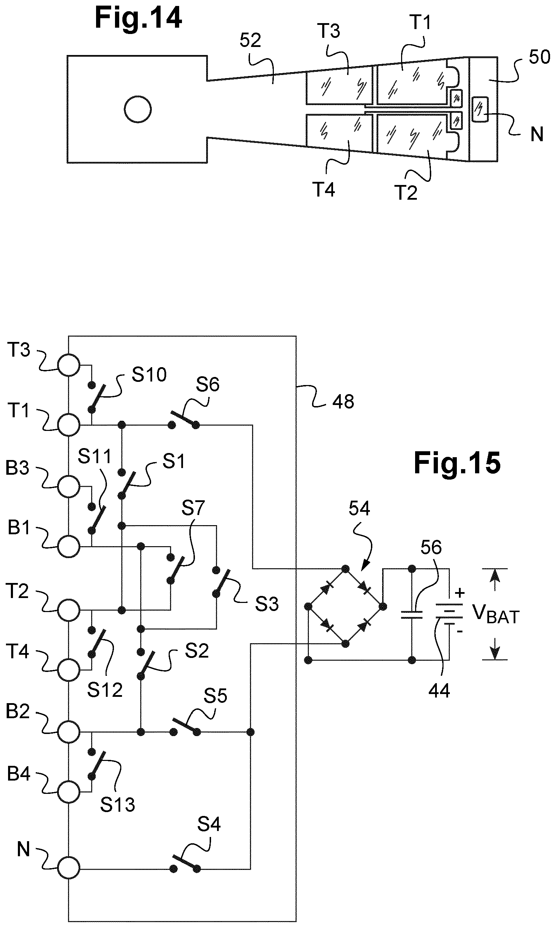

Ainsi, la Figure 14 illustre, en plan, une variante du PZT des Figures 6 et 7 où chaque électrode de surface fractionnée de la lame est elle-même subdivisée en sous-électrodes électriquement indépendantes, permettant d'augmenter encore les combinaisons de couplage par la matrice de commutation.

Dans cet exemple illustré, l'électrode *top* est divisées en quatre sous-électrodes T1, T2, T3 et T4, et de la même façon l'électrode *bottom* est divisée en quatre sous-électrodes B1, B2, B3 et B4 .

La Figure 15 est un schéma synoptique d'une matrice de commutation adaptée au PZT à sous-électrodes multiples de la Figure 14.

Cette matrice de commutation est couplée aux électrodes T1, T2, T3, T4, B1, B2, B3, B4 et N et permet de combiner entre elles ces différentes électrodes au moyen de commutateurs S1... S13 pour délivrer en sortie un signal oscillant combiné à un PMU de recharge de la batterie, par exemple constitué, comme illustré, d'un redresseur double alternance FBR 54 tel qu'un pont de Graetz et d'un condensateur de filtrage 56 couplés à la batterie 44.

L'état ouvert ou fermé des commutateurs S1... S13 permet d'obtenir sélectivement une multiplicité de configurations fonctionnelles différentes telles que :
configuration parallèle : S1, S2, S3, S4 et S6 fermés ;
configuration série : S1, S2, S5 et S6 fermés ;
configuration avec utilisation des sous-électrodes additionnelles : S5, S6 et S7 fermés ;
délivrance d'un surcroît de tension (avec puissance moindre) : S10, S11, S12 et S13 ouverts (cette configuration pouvant être combinée aux autres configurations) ;
maximisation de la puissance délivrée : S10, S11, S12 et S13 fermés (cette configuration pouvant être, ici encore, combinée aux autres configurations).

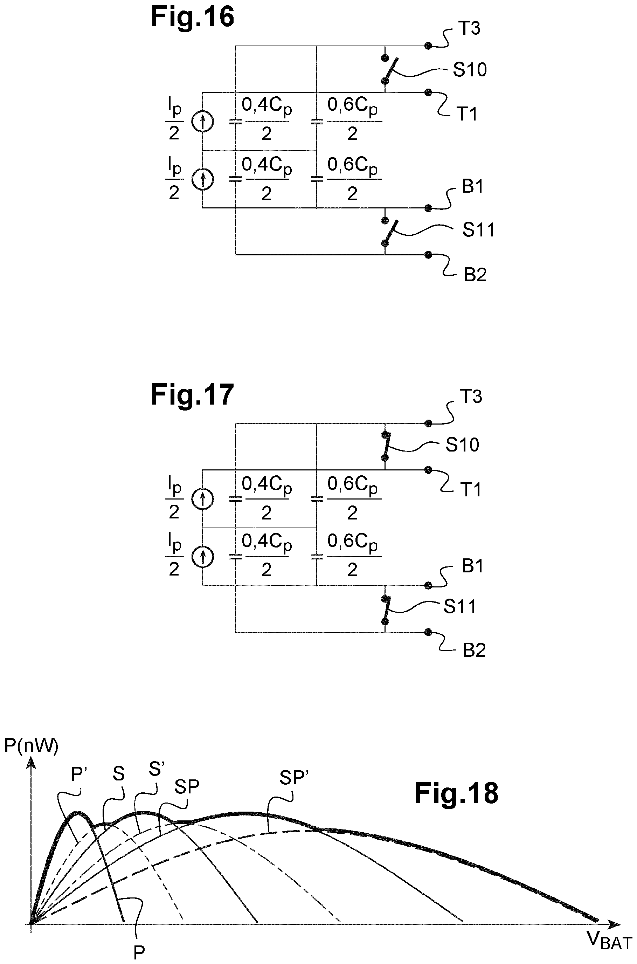

Les Figures 16 et 17 illustrent en particulier deux configurations électriques équivalentes additionnelles rendues possibles dans cet exemple, respectivement pour une ouverture et pour une fermeture des commutateurs S10 et S11 :
dans le premier cas, la configuration équivaut à une source de courant Iₚ/2 ayant une capacité interne 0,6Cₚ/4 (d'où une tension accrue du fait de la réduction de la capacité interne), tandis que
dans le second cas la configuration équivaut à une source de courant Iₚ/2 ayant une capacité interne Cₚ/4 (T1 étant en parallèle avec T3 et B1 en parallèle avec B3). La tension délivrée est moindre que dans le cas précédent, mais la puissance délivrée est supérieure.

La Figure 18 illustre six caractéristiques décrivant la variation de la puissance efficace recueillie par le PEH en fonction de la tension aux bornes de la batterie.

Par rapport à la Figure 11 décrite plus haut, la multiplication du nombre d'électrodes et donc du nombre de possibilités de commutations permet d'augmenter le nombre de caractéristiques sélectionnables : outre les configurations série S, parallèle P et série-parallèle SP décrites plus haut (représentées en trait plein), l'ouverture des commutateurs S10 et S11 crée des configurations supplémentaires correspondantes S', P' et SP' (représentées en tiretés) procurant un surcroît de tension. En fonction du niveau de tension V_{BAT} de la batterie, on pourra par des commutations appropriées sélectionner ainsi la plage offrant le meilleur compromis puissance/tension.

## Revendications

1. Un module de récupération d'énergie, comprenant :
un ensemble pendulaire (40) soumis à des sollicitations externes appliquées au module, l'ensemble pendulaire comprenant une lame (24) élastiquement déformable en flexion suivant au moins un degré de liberté, avec une extrémité encastrée et une extrémité opposée libre couplée à une masse inertielle (26),
dans lequel la lame (24) est une lame piézoélectrique formant un transducteur mécanoélectrique apte à convertir une énergie mécanique produite par des oscillations de l'ensemble pendulaire en un signal électrique oscillant (V_{OUT}(t)) recueilli par des électrodes ; et
un circuit de gestion d'alimentation (42), apte à redresser et réguler le signal recueilli par les électrodes, pour délivrer en sortie une tension ou un courant continus d'alimentation stabilisés,
***caractérisé en ce que*** la lame piézoélectrique (24) porte une pluralité d'électrodes (T1, T2, B1, B2, N) dont le nombre et la configuration définit une pluralité de paires d'électrodes entre lesquelles peuvent être recueillis simultanément une pluralité correspondante desdits signaux oscillants,
***en* ce *que*** le module de récupération d'énergie comprend en outre, entre la lame piézoélectrique et le circuit de gestion d'alimentation, une matrice de commutation (48) apte à commuter sélectivement entre elles la pluralité de paires d'électrodes selon une pluralité de configurations série (S), parallèle (P) et/ou série-parallèle (SP) différentes, pour délivrer en sortie un signal oscillant combiné appliqué au circuit de gestion d'alimentation,
*et **en ce que*** la matrice de commutation commute sélectivement en fonction d'un signal de niveau de tension (V_{BAT}) d'un organe de stockage d'énergie (44) couplé en sortie du circuit de gestion d'alimentation et rechargé par ladite tension continue d'alimentation stabilisée.

2. Le module de la revendication 1, comprenant en outre un moyen de comparaison du niveau de tension courant (V_{BAT}) de l'organe de stockage d'énergie (44) à au moins deux plages successives (I, II, III) de niveau de tension, et dans lequel une transition d'une plage à l'autre du niveau courant de la tension de l'organe de stockage d'énergie au cours de la recharge est apte à commander un changement de configuration série, parallèle et/ou série-parallèle de la matrice de commutation (48).

3. Le module de la revendication 2, dans lequel les plages successives (I, II, III) de niveau de tension sont des plages correspondant chacune à une configuration respective série (S), parallèle (P) et/ou série-parallèle (SP) maximisant la puissance restituée par la lame piézoélectrique.

4. Le module de la revendication 1, dans lequel le circuit de gestion d'alimentation est essentiellement constitué par un étage de redressement (54) et un étage de filtrage (56), lesdits étages recevant en entrée le signal oscillant combiné délivré par la matrice de commutation (48) et délivrant en sortie ladite tension ou ledit courant continus stabilisés.

5. Le module de la revendication 1, dans lequel la lame piézoélectrique (24) est une lame bimorphe comprenant une âme centrale conductrice (50) et au moins deux électrodes de surface (T1, T2 ; B1, B2) sur chaque côté de la lame, l'âme centrale conductrice et/ou les électrodes de surface étant reliées en entrée de la matrice de commutation (48) pour définir sélectivement des paires d'électrodes combinées entre elles pour produire ladite pluralité de configurations série (S), parallèle (P) et/ou série-parallèle (SP) différentes.

6. Le module de la revendication 1, dans lequel lesdites au moins deux électrodes de surface (T1, T2; B1, B2) sont subdivisées en sous-électrodes (T1, T2, T3, T4; B1, B2, B3, B4) sélectivement commutables par la matrice de commutation en complément desdites configurations série, parallèle et/ou série-parallèle, pour modifier la capacité équivalente desdites électrodes de manière à produire un surcroit de tension ou de puissance en sortie de la matrice de commutation.

7. Le module de la revendication 1, dans lequel la matrice de commutation (48) est constitués de composants commutateurs statiques (S1... S7) commandables individuellement.

8. Le module de la revendication 7, dans lequel les composants commutateurs statiques (S1... S7) comprennent chacun une paire de transistors PMOS/NMOS complémentaires (60, 62) commandés symétriquement en conduction ou en blocage.

9. Le module de la revendication 1,
dans lequel le module est incorporé à un dispositif autonome (10) logeant, dans un corps de dispositif (12) : un ensemble électronique ; ledit module de récupération d'énergie ; et un organe de stockage d'énergie (44) pour l'alimentation de l'ensemble électronique,
et dans lequel ladite tension ou ledit courant continus stabilisés délivrés par le circuit de gestion d'alimentation servent à l'alimentation de l'ensemble électronique et/ou à la recharge de l'organe de stockage d'énergie du dispositif autonome.

10. Le module de la revendication 9, dans lequel le dispositif autonome (10) est un dispositif médical actif.

11. Le module de la revendication 10, dans lequel le dispositif médical actif est une capsule autonome implantable (10) comprenant un corps de capsule (12) muni d'un élément d'ancrage (16) à une paroi d'un organe (22) d'un patient,
et dans laquelle lesdites sollicitations externes auxquelles est soumis l'ensemble pendulaire (40) du module de récupération d'énergie sont des sollicitations appliquées au corps de capsule sous l'effet de mouvements de ladite paroi et/ou de variations de débit d'un flux sanguin dans le milieu environnant.

## Patentansprüche

1. Energierückgewinnungsmodul, umfassend:
eine pendelartige Anordnung (40), die äußere Belastungen erfährt, die auf das Modul aufgebracht werden, wobei die pendelartige Anordnung ein Längsorgan (24) umfasst, das gemäß zumindest einem Freiheitsgrad beim Biegen verformbar ist und ein eingespanntes Ende und ein gegenüberliegendes freies Ende, das an eine träge Masse (26) gekoppelt ist, aufweist,
wobei das Längsorgan ein piezoelektrisches Längsorgan ist, das einen mechanisch-elektrischen Wandler bildet, der eingerichtet ist, um eine mechanische Energie, die durch Schwingungen der pendelartigen Anordnung erzeugt wird, in ein oszillierendes elektrisches Signal (V_{OUT}(t)) umzuwandeln, das von Elektroden empfangen wird; und eine Versorgungsverwaltungsschaltung (24) die eingerichtet ist, um das von den Elektroden empfangene Signal gleichzurichten und zu regeln, um am Ausgang eine stabilisierte Versorgungsgleichspannung oder einen stabilisierten Versorgungsgleichstrom auszugeben,
**dadurch gekennzeichnet, dass** das piezoelektrische Längsorgan (24) eine Mehrzahl von Elektroden (T1, T2, B1, B2, N) trägt, deren Anzahl und Konfiguration eine Mehrzahl von Elektrodenpaaren definiert, zwischen denen gleichzeitig eine entsprechende Mehrzahl der oszillierenden Signale empfangen werden können,
dass das Energierückgewinnungsmodul ferner, zwischen dem piezoelektrischen Längsorgan und der Versorgungsverwaltungsschaltung, eine Schaltmatrix (48) umfasst, die eingerichtet ist, um die Mehrzahl von Elektrodenpaaren gemäß einer Mehrzahl von unterschiedlichen seriellen (S) Konfigurationen, parallelen (P) Konfigurationen und/oder seriell-parallelen Konfigurationen (SP) selektiv untereinander zu schalten, um am Ausgang ein kombiniertes oszillierendes Signal auszugeben, das auf die Versorgungsverwaltungsschaltung aufgebracht wird,
und dass die Schaltmatrix in Abhängigkeit von einem Spannungsniveausignal (V_{BAT}) eines Energiespeicherorgans (44) selektiv schaltet, das am Ausgang der Versorgungsverwaltungsschaltung gekoppelt ist und durch die stabilisierte Versorgungsgleichspannung aufgeladen wird.

2. Modul nach Anspruch 1, ferner umfassend ein Mittel zum Vergleichen des aktuellen Spannungsniveaus (V_{BAT}) des Energiespeicherorgans (44) mit wenigstens zwei aufeinander folgenden Spannungsniveaubereichen (I, II, III) und wobei ein Übergang von einem aktuellen Niveau der Spannung des Energiespeicherorgans zu einem anderen im Laufe der Aufladung eingerichtet ist, um eine Änderung der seriellen Konfiguration, parallelen Konfiguration und/oder seriell-parallelen Konfiguration zu steuern.

3. Modul nach Anspruch 2, wobei die aufeinander folgenden Spannungsniveaubereiche (I, II, III) Bereiche sind, die jeweils einer jeweiligen seriellen (S) Konfiguration, parallelen (P) Konfiguration und/oder seriell-parallelen Konfiguration (SP) entsprechen, welche die durch das piezoelektrische Längsorgan zurückgegebene Leistung maximieren.

4. Modul nach Anspruch 1, wobei die Versorgungsverwaltungsschaltung im Wesentlichen von einer Gleichrichterstufe (54) und einer Filterstufe (56) gebildet ist, wobei die Stufen am Eingang das von der Schaltmatrix (48) ausgegebene kombinierte oszillierende Signal empfangen und am Ausgang die stabilisierte Gleichspannung oder den stabilisierten Gleichstrom ausgeben.

5. Modul nach Anspruch 1, wobei das piezoelektrische Längsorgan (24) ein bimorphes Längsorgan ist, das eine leitende mittige Seele (50) und wenigstens zwei Oberflächenelektroden (T1, T2; B1, B2) auf jeder Seite des Längsorgans umfasst, wobei die leitende mittige Seele und/oder die Oberflächenelektroden am Eingang der Schaltmatrix (48) verbunden sind, um miteinander kombinierte Elektrodenpaare selektiv zu definieren, um die Mehrzahl von unterschiedlichen seriellen (S) Konfigurationen, parallelen (P) Konfigurationen und/oder seriell-parallelen Konfiguration (SP) zu erzeugen.

6. Modul nach Anspruch 1, wobei die wenigstens zwei Oberflächenelektroden (T1, T2; B1, B2) unterteilt sind in Teilelektroden (T1, T2, T3, T4; B1, B2, B3, B4), die durch die Schaltmatrix zusätzlich zu den seriellen Konfigurationen, parallelen Konfigurationen und/oder seriell-parallelen Konfiguration selektiv schaltbar sind, um die äquivalente Kapazität der Elektroden so zu verändern, dass eine zunehmende Spannung oder Leistung am Ausgang der Schaltmatrix erzeugt wird.

7. Modul nach Anspruch 1, wobei die Schaltmatrix (48) von statischen Schaltbauteilen (Sl... S7) gebildet ist, die einzeln ansteuerbar sind.

8. Modul nach Anspruch 7, wobei die statischen Schaltbauteile (Sl... S7) jeweils ein Paar komplementäre PMOS/NMOS-Transistoren (60, 62) umfassen, die symmetrisch zur Leitung oder zur Sperrung angesteuert werden.

9. Modul nach Anspruch 1,
wobei das Modul in eine autonome Vorrichtung (10) eingebettet ist, die eine elektronische Einheit, das Energierückgewinnungsmodul, und ein Energiespeicherorgan (44) für die Versorgung der elektronischen Einheit in einem Vorrichtungskörper (12) aufnimmt,
und wobei die stabilisierte Gleichspannung oder der stabilisierte Gleichstrom, die/der von der Versorgungsverwaltungsschaltung ausgegeben wird, der Versorgung der elektronischen Einheit und/oder der Aufladung des Energiespeicherorgans der autonomen Vorrichtung dienen.

10. Modul nach Anspruch 9, wobei die autonome Vorrichtung (10) eine aktive medizinische Vorrichtung ist.

11. Modul nach Anspruch 10, wobei die aktive medizinische Vorrichtung eine implantierbare autonome Kapsel (10) ist, die einen Kapselkörper (12) umfasst, der mit einem Element (16) zur Verankerung an einer Wand eines Organs (22) eines Patienten versehen ist,
und wobei die äußeren Belastungen, welche die pendelartige Anordnung (40) des Energierückgewinnungsmoduls erfährt, Belastungen sind, die auf den Kapselkörper unter der Einwirkung von Bewegungen der Wand und/oder von Veränderungen der Blutflussrate im umgebenden Medium aufgebracht werden.

## Claims

1. An energy harvesting module, comprising:
a pendular unit (40) subjected to external stresses applied to the module, the pendular unit comprising a beam (24) that is elastically deformable in bending according to at least one degree of freedom, with a clamped end and an opposite free end coupled to an inertial mass (26),
wherein the beam (24) is a piezoelectric beam forming a mechanical-electrical transducer adapted to convert a mechanical energy produced by oscillations of the pendular unit into an oscillating electrical signal (V_{OUT}(t)) collected by electrodes; and
a power management circuit (42), adapted to rectify and regulate the signal collected by the electrodes, to output a stabilized direct power voltage or current,
***characterized in that*** the piezoelectric beam (24) carries a plurality of electrodes (T1, T2, B1, B2, N) whose number and configuration define a plurality of pairs of electrodes between which a corresponding plurality of oscillating signals can be simultaneously collected,
***in that*** the energy harvesting module further comprises, between the piezoelectric beam and the power management circuit, a switching matrix (48) adapted to selectively switch the plurality of pairs of electrodes between each other according to a plurality of different series (S), parallel (P) and/or series-parallel (SP) configurations, to output a combined oscillating signal applied to the power management circuit,
*and **in that*** the switching matrix selectively switches as a function of a voltage level signal (V_{BAT}) of an energy storage component (44) coupled to the power management circuit output and charged by said stabilized direct power voltage.

2. The module of claim 1, further comprising a means for comparing the current voltage level (V_{BAT}) of the energy storage component (44) with at least two successive voltage level ranges (I, II, III), and wherein a transition of the current voltage level of the energy storage component from one range to another during the charge is operable to control a change of series, parallel and/or series-parallel configuration of the switching matrix (48).

3. The module of claim 2, wherein the successive voltage level ranges (I, II, III) are ranges each corresponding to a respective series (S), parallel (P) and/or series-parallel (SP) configuration maximizing the power returned by the piezoelectric beam.

4. The module of claim 1, wherein the power management circuit is essentially consisted of a rectification stage (54) and a filtering stage (56), said stages receiving as an input the combined oscillating signal output by the switching matrix (48) and outputting said stabilized direct voltage or current.

5. The module of claim 1, wherein the piezoelectric beam (24) is a bimorphous beam comprising a central conductive shim (50) and at least two surface electrodes (T1, T2 ; B1, B2) on each side of the beam, the central conductive shim and/or the surface electrodes being connected to the input of the switching matrix (48) to selectively define pairs of electrodes combined to each other to produce said plurality of different series (S), parallel (P) and/or series-parallel (SP) configurations.

6. The module of claim 1, wherein said at least two surface electrodes (T1, T2 ; B1, B2) are subdivided into sub-electrodes (T1, T2, T3, T4 ; B1, B2, B3, B4) that are selectively switchable by the switching matrix in complement to said series, parallel and/or series-parallel configurations, to modify the equivalent capacitance of said electrodes in order to produce an extra voltage or power at the switching matrix output.

7. The module of claim 1, wherein the switching matrix (48) is consisted of individually controllable static switching components (S1... S7).

8. The module of claim 7, wherein the static switching components (S1... S7) each comprise a pair of complementary PMOS/NMOS transistors (60, 62) that are symmetrically controlled in conduction or cutoff state.

9. The module of claim 1,
wherein the module is incorporated to an autonomous device (10) which houses, within a device body (12): an electronic unit; said energy harvesting module; and an energy storage component (44) for powering the electronic unit,
and wherein said stabilized direct voltage or current output by the power management circuit is to power the electronic unit and/or to charge the energy storage component of the autonomous device.

10. The module of claim 9, wherein the autonomous device (10) is an active medical device.

11. The module of claim 10, wherein the active medical device is an autonomous implantable capsule (10) comprising a capsule body (12) provided with an anchoring element (16) for its anchoring to a wall of an patient's organ (22),
and wherein said external stresses to which is subjected the pendular unit (40) of the energy harvesting module are stresses applied to the capsule body under the effect of movements of said wall and/or blood flow rate variations in the surrounding environment.
